# FASCICULE DE BREVET EUROPEEN

(11) **EP 2 685 916 B1**
(45) Date de publication et mention de la délivrance du brevet: **11.10.2017**
(21) Numéro de dépôt: 12710482.6
(22) Date de dépôt: 16.03.2012
(51) Int. Cl.: A61B 17/17

(54) **INSTRUMENTATION CHIRURGICALE SPÉCIFIQUE D'UN PATIENT POUR LA PRÉPARATION DU FÉMUR DE CE PATIENT**
PATIENTENSPEZIFISCHES CHIRURGISCHES INSTRUMENTARIUM ZUR FEMURVORBEREITUNG BEI EINEM PATIENTEN
PATIENT-SPECIFIC SURGICAL INSTRUMENTATION FOR PREPARING THE PATIENT'S FEMUR

(30) Priorité: 17.03.2011 US 201161453879 P; 26.07.2011 FR 1156803
(43) Date de publication de la demande: 22.01.2014
(73) Titulaire: Corin Limited, The Corinium Centre Cirencester GL7 1YJ (GB)
(72) Inventeur: ZAKARIA, Toufik, F-38000 Grenoble (FR); HENRY, Delphine, F-38330 Saint-ismier (FR)
(74) Mandataire: Hocking, Adrian Niall
(86) Numéro de dépôt international: PCT/EP2012/054701
(87) Numéro de publication internationale: WO 2012/123580

(56) Documents cités:
- WO-A1-2006/069336
- WO-A1-2009/001083
- US-A1- 2004 220 583
- US-A1- 2007 173 851

## Description

La présente invention concerne une instrumentation chirurgicale spécifique à un patient permettant de préparer le fémur de ce patient, typiquement en vue d'y implanter un composant fémoral d'une prothèse de genou.

Ainsi, l'invention porte sur une instrumentation fémorale que l'on qualifie également de « sur-mesure » ou personnalisée, en lien avec un patient précis, exclusivement sur lequel l'instrumentation est destinée à être utilisée. Ce genre d'instrumentation spécifique à un patient s'oppose aux instrumentations standards, qui sont utilisées indifféremment sur divers patients, le cas échéant en étant réutilisées plusieurs fois de manière successive, en étant nettoyées et stérilisées entre chaque utilisation.

L'avènement des instrumentations « sur-mesure » est lié aux possibilités actuelles d'acquérir des données pré-opératoires suffisamment précises afin de concevoir, notamment du point de vue dimensionnel, des instruments dont les interfaces de coopération mécanique avec les os du patient sont spécifiquement définies en tenant compte de la forme précise, notamment des reliefs de surface, de ces os. Les données pré-opératoires utilisées proviennent typiquement d'images scanner ou, plus généralement, de tout enregistrement de données de cartographie osseuse avantageusement obtenues de manière non invasive. Ces données sont traitées par ordinateur afin de commander la fabrication d'instruments chirurgicaux sur mesure, une fois que le chirurgien a décidé les détails de la procédure chirurgicale qu'il va suivre pas-à-pas lors d'une intervention à venir.

Dans ce contexte, l'invention s'intéresse plus spécifiquement aux instrumentations chirurgicales sur mesure destinées à préparer l'extrémité inférieure du fémur d'un patient, typiquement aux fins d'implantation du composant fémoral d'une prothèse de genou, étant remarqué que cette dernière peut aussi bien être une prothèse également sur mesure, c'est-à-dire personnalisée spécifiquement au patient à opérer, qu'une prothèse de genou « de catalogue », c'est-à-dire une prothèse standard, produite en série, le cas échéant déclinée en gammes dimensionnelles. Dans le contexte de la préparation de l'extrémité inférieure du fémur, le recours à une instrumentation spécifique au patient à opérer présente un réel intérêt, du fait de la complexité de l'articulation du genou et de la nécessité de préparer l'os du fémur avec la plus grande précision possible, dans le sens où cette préparation détermine directement et significativement le positionnement d'implantation du composant fémoral sur le fémur, ainsi que vis-à-vis du tibia : on comprend donc que les performances mécaniques ultérieures de la prothèse implantée sont directement liées à la meilleure implantation possible, en ce qui concerne le positionnement des composants prothétiques vis-à-vis du fémur et du tibia.

En pratique, dans le contexte évoqué juste ci-dessus, les instrumentations sur mesure actuelles consistent généralement en un bloc monolithique, qui, comme expliqué plus haut, a été fabriqué en utilisant des données de cartographie osseuse pré-opératoires, relatives à un patient précis à opérer, et que le chirurgien utilise spécifiquement sur ce patient : après avoir mis en place ce bloc sur l'extrémité inférieure du fémur, selon une configuration unique prédéterminée, liée à la coopération par complémentarité de formes entre une surface d'appui, délimitée par ce bloc, et l'extrémité inférieure du fémur, le chirurgien utilise ce guide pour contrôler l'application d'un ou de plusieurs outils de préparation osseuse, tels qu'un foret de perçage ou une broche d'ancrage. Ce ou ces outils permettent alors au chirurgien de préparer l'extrémité du fémur, notamment permettent de réséquer cette extrémité du fémur selon un ou plusieurs plans géométriques précis, ces plans de coupe étant notamment dimensionnés pour former des appuis plans correspondants pour la fixation d'un composant fémoral prothétique. Par nature, ce bloc de guidage, spécifique au patient à opérer, ne laisse au chirurgien aucune possibilité d'ajustement quant à l'application des outils de préparation osseuse précités : en effet, le recours à un tel bloc de guidage sur mesure vise, justement, à faciliter et sécuriser les gestes chirurgicaux, ces derniers étant réalisés, au cours de l'intervention, en suivant un planning opératoire prédéterminé par le chirurgien, notamment sur la base des données de cartographie osseuse pré-opératoires. En théorie, cette approche contrainte, à sens unique, garantit un résultat implantatoire optimal. Toutefois, dans la pratique, les chirurgiens constatent fréquemment que, notamment en raison de l'environnement ligamentaire de l'articulation du genou, le planning opératoire pré-décidé peut ne pas être totalement satisfaisant en ce qui concerne le positionnement d'implantation du composant prothétique fémoral, eu égard à ce qui est couramment appelé la rotation externe-interne du fémur, c'est-à-dire à la position angulaire du fémur autour de son axe longitudinal. En effet, alors que cette rotation du fémur a été correctement quantifiée à l'aide des données pré-opératoires, il arrive fréquemment que les actions chirurgicales réalisées au début de la procédure d'implantation entraînent une modification, même légère, de cette valeur pré-estimée de la rotation externe-interne du fémur. Plus généralement, en cours d'intervention, certains chirurgiens estiment qu'il aurait été préférable de s'écarter, même légèrement, du planning opératoire pré-décidé. Toutefois, de tels ajustements per-opératoires sont impossible à mettre en oeuvre avec le bloc de guidage sur mesure décrit plus haut.

Par ailleurs, WO-A-2011/029911, qui a été publié le jour de la priorité la plus ancienne revendiquée par la présente demande, propose une instrumentation dont le bloc fémoral, spécifique à un patient dont le fémur est à préparer, porte un système mécanique d'ajustement à la rotation varus-valgus du genou du patient, laquelle rotation varus-valgus caractérise, genou en extension, la position angulaire relative entre le fémur et le tibia dans un plan contenant les axes longitudinaux respectifs du fémur et du tibia (comme bien montré à la figure 5 de WO-A-2011/029911). L'ajustement réalisé n'est pas libre, mais est contraint sous l'action d'éléments élastiques qui, en service, sont déformés en se trouvant interposés mécaniquement entre le tibia et le bloc spécifique fixé au fémur.

Le document WO 2009/001083 A1, qui est considéré comme l'état de la technique le plus proche, décrit une instrumentation chirurgicale conforme au préambule de la revendication 1. Le but de la présente invention est de proposer une instrumentation sur mesure de préparation du fémur d'un patient, qui permet au chirurgien de tenir compte, lors de l'intervention chirurgicale proprement dite, de la rotation externe-interne du fémur.

A cet effet, l'invention a pour objet une instrumentation chirurgicale spécifique à un patient, pour la préparation du fémur de ce patient, telle que définie à la revendication 1.

Une des idées à la base de l'invention est d'associer à un bloc fémoral sur mesure un système mécanique permettant au chirurgien de tenir compte de la rotation externe-interne du fémur, notamment pour ajuster, en per-opératoire, l'application d'un outil de préparation osseuse. Ainsi, contrairement à la « philosophie » traditionnelle d'une instrumentation sur mesure, l'instrumentation selon une forme de l'invention inclut avantageusement une pièce de guidage, qui, en service, est portée par un bloc fémoral, spécifique à un patient, et qui est à même de tourner, par rapport au bloc, autour d'un axe de rotation qui, en service, s'étend selon la direction longitudinale du fémur du patient opéré. Moyennant le réglage de la position angulaire de cette pièce de guidage autour de l'axe de rotation, le chirurgien peut donc ajuster l'application de l'outil de préparation à la rotation externe-interne du fémur qu'il constate au cours de l'intervention chirurgicale. Bien entendu, eu égard à la nature du mouvement du fémur dont on cherche à tenir compte grâce à l'instrumentation selon l'invention, c'est-à-dire de la rotation externe-interne du fémur, on comprend que le système d'ajustement est, en service, libre vis-à-vis des autres os du genou, notamment vis-à-vis du tibia.

Des caractéristiques additionnelles avantageuses de l'instrumentation conforme à l'invention, prises isolément ou selon toutes les combinaisons techniquement possibles, sont spécifiées aux revendications 2 à 15.

L'invention sera mieux comprise à la lecture de la description qui va suivre, donnée uniquement à titre d'exemple et faite en se référant aux dessins sur lesquels :
- la figure 1 est une vue en élévation d'une partie d'un premier mode de réalisation d'une instrumentation conforme à l'invention, en cours d'utilisation sur un fémur, la direction d'observation de cette figure 1 correspondant à la direction longitudinale du fémur ;
- la figure 2 est une coupe selon la ligne II-II de la figure 1 ;
- la figure 3 est une vue analogue à la figure 1, montrant des parties additionnelles du mode de réalisation de la figure 1, également en cours d'utilisation sur le fémur ;
- la figure 4 est une coupe selon la ligne IV-IV de la figure 3 ;
- la figure 5 est une vue en élévation selon la flèche V de la figure 3 ;
- la figure 6 est une vue en perspective d'une partie d'un second mode de réalisation d'une instrumentation conforme à l'invention, en cours d'utilisation sur un fémur, l'étape d'utilisation illustrée par la figure 6 étant similaire à l'étape d'utilisation illustrée à la figure 1 ;
- la figure 7 est une vue analogue à la figure 6, montrant une partie additionnelle du second mode de réalisation de l'instrumentation de la figure 6 ;
- la figure 8 est une vue analogue à la figure 7, montrant encore une partie additionnelle du second mode de réalisation de l'instrumentation de la figure 6 ; et
- la figure 9 est une vue en élévation selon la flèche IX de la figure 8.

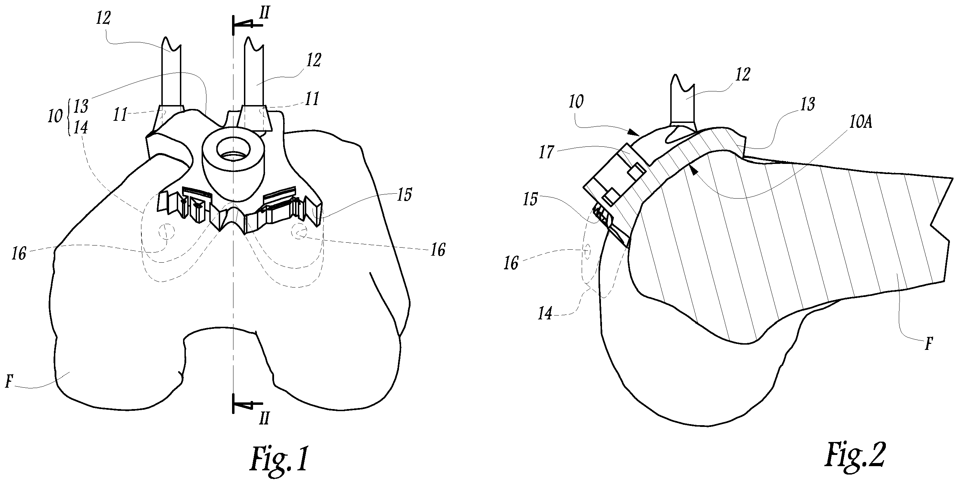

Sur les figures 1 à 5 est représentée une instrumentation chirurgicale 1 comprenant plusieurs composants qui vont être détaillés les uns après les autres, au fur et à mesure de la description d'une utilisation de cette instrumentation sur le fémur F d'un patient, typiquement pour la préparation de l'extrémité inférieure de ce fémur à l'implantation d'un composant fémoral d'une prothèse de genou, non représentée.

Dans toute la suite, les termes « supérieur », « inférieur », « postérieur », etc. s'entendent dans leur sens anatomique usuel, en considérant que le patient opéré se tient debout sur une surface horizontale.

Préalablement à l'intervention chirurgicale d'implantation proprement dite, on recueille des données de cartographie relatives au fémur F du patient à opérer. En pratique, ces données de cartographie pré-opératoires peuvent être obtenues de diverses manières. A titre d'exemple, des images scanner et/ou radiographiques et/ou échographies et/ou IRM du fémur F sont utilisées.

Dans tous les cas, à l'issue de cette étape préalable d'acquisition des données, on dispose de suffisamment d'informations pour concevoir et fabriquer un bloc fémoral 10 spécifique au patient, montré seul en position sur le fémur sur les figures 1 et 2. Plus précisément, le bloc fémoral 10 présente une surface 10A qui est conformée de manière spécifiquement ajustée à l'extrémité inférieure du fémur F et qui, en service, est appuyée fixement contre cette extrémité du fémur, en épousant la surface de cette dernière par complémentarité de formes. On comprend que, pour aboutir à un tel ajustement rigoureux entre la surface d'appui 10A et l'extrémité inférieure du fémur F, la surface 10A est dessinée en utilisant les données de cartographie pré-opératoires relatives au fémur. De la sorte, la surface d'appui 10A présente des reliefs personnalisés spécifiquement au patient opéré qui, en coopérant avec des reliefs complémentaires délimités par la surface de l'extrémité inférieure du fémur, n'autorisent qu'une seule configuration d'appui ajusté sur le fémur F, comme représenté sur les figures 1 et 2. A titre d'exemple, dans le mode de réalisation considéré sur les figures 1 et 2, la surface d'appui 10A recouvre des zones antérieure et distale de l'extrémité inférieure du fémur F, en épousant de manière ajustée les reliefs de ces zones.

En service, le bloc fémoral 10 est destiné à être appuyé fixement, par sa surface 10A, sur l'extrémité inférieure du fémur F. Dans l'exemple de réalisation considéré, cette fixation est réalisée grâce à, d'une part, deux trous traversants 11, reliant la surface 10A à la face opposée, c'est-à-dire celle tournée vers le chirurgien, du bloc 10 et, d'autre part, deux broches d'ancrage osseux 12 à même d'être respectivement engagées de façon complémentaire dans les trous traversants 11, comme représenté sur les figures 1 et 2, jusqu'à se planter et ainsi s'immobiliser dans la matière osseuse du fémur.

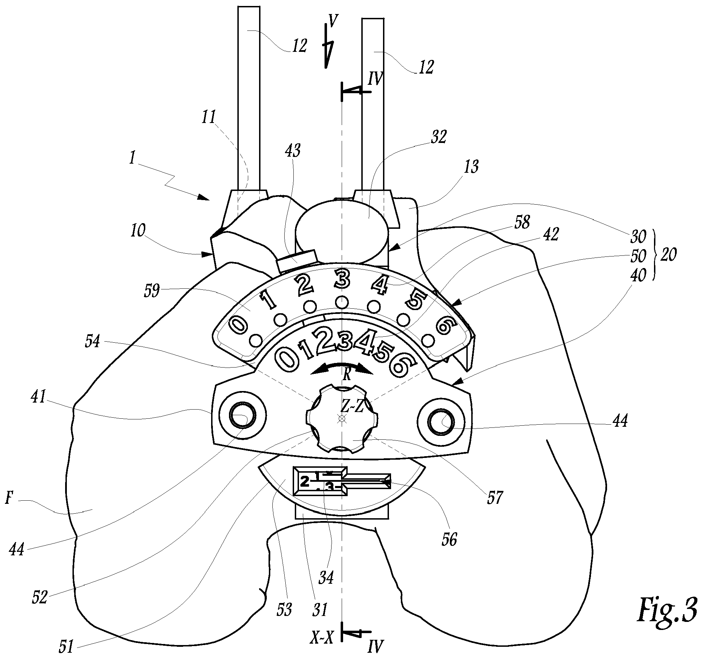

Sur les figures 1 et 2, on remarquera que le bloc fémoral 10 est représenté pour partie seulement en traits pleins et, pour le reste, en traits pointillés. Cette représentation vise à faire comprendre que le bloc fémoral 10 est constitué de deux parties ayant des finalités respectives différentes, comme expliqué progressivement ci-après, à savoir une partie principale 13 et une partie détachable 14. La partie principale 13, représentée en traits pleins, est celle pourvue des trous traversant 11 et délimitant la majeure partie, voire la quasi totalité de la surface d'appui 10A. Cette partie principale 13 est destinée à être présente pendant toute l'utilisation de l'instrumentation 1, tandis que la partie détachable 14 est, au contraire, conçue pour pouvoir être séparée, par le chirurgien, de la partie principale 13. En pratique, dans la mesure où le bloc fémoral 10 est mis à disposition du chirurgien sous une forme initialement monolithique, on comprend que la séparation de la partie 14 vis-à-vis de la partie principale 13 nécessite pour le chirurgien de rompre le lien de matière entre les parties 13 et 14, ce qui est représenté sur les figures 1 et 2 par la ligne de brisure 15. En fait, la présence temporaire, au cours de l'intervention, de la partie 14 vis-à-vis du reste du bloc 10 permet de bien comprendre l'intérêt de la présente invention. En effet, la partie détachable 14 correspond à la partie du bloc 10 qui a été conçue, de manière pré-opératoire, pour guider l'application per-opératoire sur le fémur F d'un outil de préparation osseuse, non représenté sur les figures, tel qu'un foret de perçage ou une broche d'ancrage. D'ailleurs, sur les figures 1 et 2, la partie détachable 14 est pourvue de deux trous traversants 16, eux aussi représentés en pointillés : lors de la conception sur mesure du bloc 10, les trous 16 ont été positionnés au sein du bloc 10 pour guider, de manière non ajustable, c'est-à-dire sans liberté possible pour le chirurgien, le positionnement de moyens de coupe du fémur, tels qu'un bloc de coupe, afin de réséquer l'extrémité du fémur selon un ou plusieurs plans de résection dimensionnés pour former ultérieurement des appuis plans correspondants pour la fixation d'un composant prothétique fémoral. Comme expliqué dans la partie introductive du présent document, l'utilisation des trous 16 contraindrait le chirurgien à préparer le fémur F rigoureusement comme planifié en pré-opératoire, sans avoir la possibilité de tenir compte de la rotation externe-interne du fémur F que le chirurgien peut constater de manière per-opératoire, précisément juste après avoir incisé les chaires molles entourant l'articulation du genou en flexion, afin d'accéder à l'extrémité inférieure du fémur F, et avoir fixé sur le fémur F le bloc 10. L'instrumentation 1 répond à cette problématique, d'une part, en permettant au chirurgien de dégager la partie détachable 14 et, d'autre part, en utilisant un système mécanique d'ajustement 20, qui va être décrit ci-après en détail en regard des figures 3 à 5. En pratique, on notera que le dégagement de la partie détachable 14 permet avantageusement de libérer de l'espace en regard de la zone du fémur F sur laquelle débouchaient les trous 16, à savoir la zone distale de l'extrémité inférieure du fémur F, cet espace ainsi libéré étant ensuite au moins partiellement occupé par le système mécanique 20, comme bien visible sur les figures 3 à 5.

Comme représenté sur les figures 3 à 5, le système mécanique 20 se présente sous la forme d'un ensemble pré-assemblé de trois composants appartenant à l'instrumentation 1, à savoir un support 30, une pièce 40 de guidage de l'application d'un outil de préparation osseuse, tel qu'un foret de perçage ou une broche d'ancrage, et une pièce 50 interposée mécaniquement entre le support 30 et la pièce de guidage 40 afin de les relier l'un à l'autre.

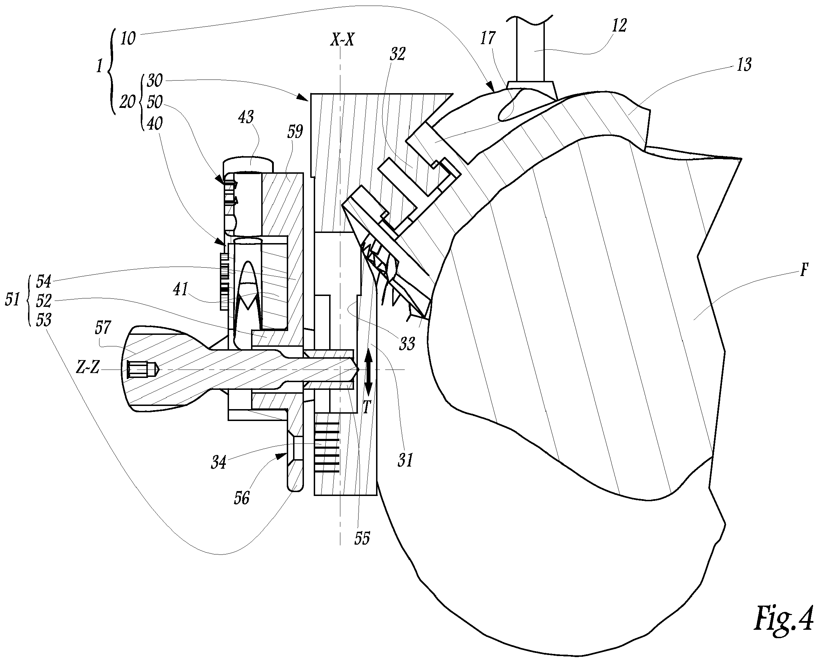

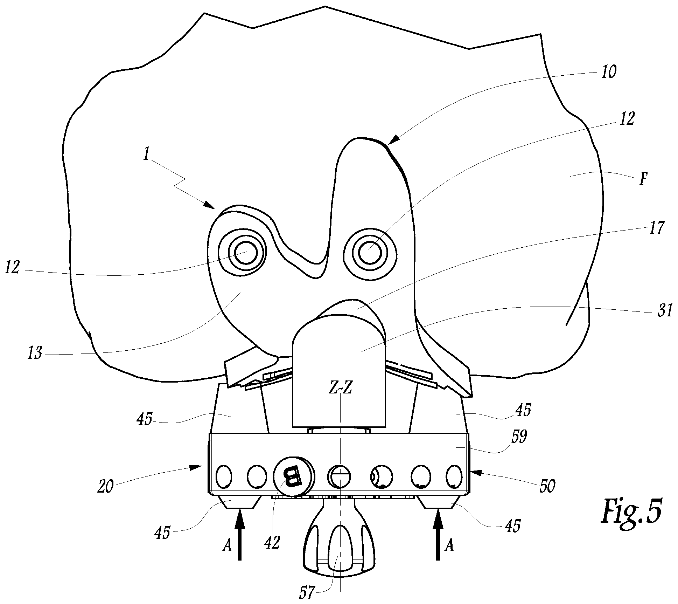

Plus précisément, le support 30 comporte un corps rigide allongé 31, qui s'étend en longueur suivant un axe géométrique central X-X qui est contenu dans le plan de coupe IV-IV. A l'une de ses extrémités axiales, qui est tournée vers le haut en service, le corps 31 est pourvu d'un plot 32 de fixation sur la partie principale 13 du bloc fémoral 10. Comme bien visible sur les figures 4 et 5, ce plot 32 est adapté pour s'engager à l'intérieur de et ainsi mettre en prise un collier de couplage 17 dont est pourvue la partie principale 13 du bloc 10, sur sa face opposée à la surface d'appui 10A. En pratique, la forme de réalisation du plot 32 et du collier 17 n'est pas limitative de la présente invention, du moment que, plus généralement, le corps 31 est à même d'être immobilisé mécaniquement sur une région dédiée de la partie principale 13 du bloc 10.

Comme visible uniquement à la figure 4, la partie courante du corps 31 délimite, sur sa face, qui en service est tournée à l'opposé du fémur F, une rainure rectiligne 33 qui s'étend en longueur suivant l'axe X-X. Par ailleurs, à son extrémité opposée à celle munie du plot 32, le corps 31 porte, sur sa face qui en service est tournée à l'opposé du fémur F, des graduations 34, qui sont réparties de manière régulière suivant l'axe X-X et qui, dans l'exemple de réalisation considéré ici, consistent respectivement en des fentes non traversantes, parallèles entre elles et perpendiculaires à l'axe X-X, comme visible sur les figures 3 et 4. En variante non représentée, la partie du corps 31 qui porte les graduations 34 est détachable du reste du corps.

Comme représenté en pointillés sur la figure 3 et comme visible sur la figue 4, la pièce interposée 50 comprend principalement un corps rigide 51 qui inclut un moyeu central 52, centré sur un axe géométrique Z-Z. Le corps 51 inclut également deux portions de disque 53 et 54, qui sont centrées sur l'axe Z-Z et qui s'étendent depuis le moyeu 52, de manière diamétralement opposée par rapport à l'axe Z-Z. Ce corps 51 est pourvu, sur sa face tournée en service vers le support 30, d'un pion saillant 55, centré sur l'axe Z-Z. Ce pion 55 est adapté, moyennant un dimensionnement adéquat, pour coulisser à l'intérieur de la rainure 33, en suivant une trajectoire exclusivement rectiligne, selon l'axe X-X. En pratique, ce pion 55 est assemblé au corps 31 du support 30, en étant introduit à l'intérieur de la rainure 33 via l'extrémité supérieure de cette rainure, qui présente un dimensionnement transversal supérieur au reste de la rainure, pour autoriser l'insertion, suivant l'axe Z-Z, du pion 55 jusqu'à proximité du fond de la rainure 33, tandis que, une fois que ce pion est coulissé axialement dans la rainure 33, en direction opposée à cette extrémité supérieure, son dégagement axial suivant l'axe Z-Z est empêché par coopération de complémentarité de formes entre des aménagements ad hoc de la rainure 33 et du pion 55. Les axes X-X et Z-Z se trouvent agencés à la perpendiculaire l'un de l'autre. Bien entendu, à titre de variantes non représentées, d'autres montages mécaniques sont envisageables entre le corps 51 de la pièce interposée 50 et le corps 31 du support 30, du moment que, lorsque la pièce interposée 50 est montée sur le support 30, le corps 51 est guidé en translation selon l'axe X-X, de manière coulissante selon cet axe, comme indiqué par la flèche T à la figure 4, tout en définissant l'axe Z-Z à la perpendiculaire de l'axe X-X.

On comprend alors l'intérêt des graduations 34 du support 30. En effet, comme bien visible sur les figures 3 et 4, la portion de disque 53 du corps 51 est pourvue d'une fenêtre traversante 56 reliant les deux faces de cette portion de disque, opposées suivant la direction de l'axe Z-Z. A l'état assemblé du support 30 et de la pièce interposée 50, cette fenêtre 56 est en regard, suivant la direction de l'axe Z-Z, des graduations 34, permettant ainsi au chirurgien de lire les graduations, parmi les graduations 34, qui sont visibles à travers la fenêtre 56, en particulier de lire la graduation parmi elles qui est située au même niveau axial, suivant la direction de l'axe X-X, qu'une référence prédéterminée appartenant à cette fenêtre 56. Ainsi, grâce à la lecture des graduations 34 à travers la fenêtre 56, le chirurgien peut quantifier la position translatée de la pièce 50 le long de l'axe X-X. L'intérêt chirurgical de cet aménagement sera expliqué un peu plus loin.

Avantageusement, la pièce interposée 50 est associée à une mollette 57, mobile en rotation sur elle-même autour de l'axe Z-Z et engagée à travers le moyeu 52 et le pion 55, comme bien visible sur la figure 4. En pratique, comme dans l'exemple considéré ici, cette mollette 57 porte extérieurement un filetage complémentaire d'un taraudage intérieur du pion 55. A son extrémité axiale tournée en service vers le chirurgien, la mollette 57 se termine par un bouton d'entraînement en rotation, tandis qu'à son extrémité axiale opposée, la mollette 57 se termine en une pointe d'appui contre le fond de la rainure 33. Ainsi, on comprend que, moyennant le vissage de la mollette 57, cette dernière peut être appuyée fixement contre le fond de la rainure 33, et ainsi bloquer en position la pièce 50 le long de l'axe de translation X-X, par rapport au support 30.

La pièce de guidage 40 comporte, quant à elle, un corps rigide 41 présentant globalement la forme d'un disque tronqué, centré sur l'axe Z-Z. Ainsi, comme bien visible sur les figures 3 et 4, ce corps 41 est monté sur le corps 51 de la pièce 50, en étant engagé, de manière complémentaire, autour du moyeu 52, de sorte que le corps 41 recouvre l'essentiel de la portion de disque 54, tout en laissant découverte, en raison de sa troncature, la portion de disque 53, laissant ainsi visible la fenêtre 56 pour le chirurgien. Par basculement rotatif, autour de l'axe Z-Z, du corps 41 autour du moyeu 52, on comprend que la pièce 40 est assemblée à la pièce interposée 50 et au support 30 de manière mobile autour de l'axe de rotation Z-Z, comme indiqué par la flèche R à la figure 3.

Afin de quantifier le positionnement angulaire relatif, autour de l'axe Z-Z, entre la pièce de guidage 40 et la pièce interposée 50, cette dernière est pourvue de graduations 58 réparties de manière uniforme suivant une direction périphérique autour de l'axe Z-Z. Dans l'exemple de réalisation considéré sur les figures, ces graduations 58 sont portées par une bordure 59 de la portion de disque 54, courant le long de la périphérie de cette portion de disque. Avantageusement, la pièce de guidage 40 ne porte pas une référence unique, vis-à-vis de laquelle le chirurgien pourrait lire l'une des graduations 58, mais porte une pluralité de références 42, qui sont respectivement associées de manière univoque avec l'une des graduations 58 et qui s'alignent, suivant une direction radiale à l'axe Z-Z, respectivement avec leur graduation associée parmi les graduations 58 uniquement pour des positions angulaires respectives différentes. La facilité des mesures de la position angulaire de la pièce de guidage 40 s'en trouve renforcée.

De plus, la pièce de guidage 40 est associée à une tige 43 de blocage de la pièce de guidage en rotation autour de l'axe Z-Z, par rapport à la pièce 50. Plus généralement, cette tige est une forme de réalisation de moyens mécaniques permettant de bloquer, de manière réversible, le positionnement angulaire rotatif, autour de l'axe Z-Z, entre les pièces 40 et 50. Avantageusement, dans l'exemple de réalisation considéré sur les figures, cette tige 43 est introduite à travers les pièces 40 et 50 suivant une direction radiale à l'axe Z-Z, plus précisément à travers, successivement, la bordure 59 de la portion de disque 54 et la partie périphérique discoïdale du corps 41, agencée radialement en regard de cette bordure 59, comme bien visible sur les figures 3 et 5. De la sorte, en associant des passages d'insertion de la tige 43 à travers la bordure 59 et le corps 41, à chaque couple constitué d'une des graduations 58 et de sa référence associée parmi les référence 42, la tige 43 a pour fonction additionnelle de forcer l'alignement radial de la graduation et de la référence du couple concerné, sécurisant ainsi la lecture de la quantification de la position angulaire de la pièce de guidage 40 autour de l'axe de rotation Z-Z.

Comme bien visible sur la figure 3, le corps 41 de la pièce de guidage 40 est par ailleurs pourvu de deux trous traversants 44, centrés sur des axes géométriques respectifs qui sont parallèles à l'axe Z-Z et qui, dans l'exemple de réalisation, sont diamétralement opposés l'un à l'autre par rapport à cet axe Z-Z. De manière similaire à ce qui a été décrit plus haut concernant les trous traversants 16, les trous traversants 44 sont adaptés pour guider l'application d'un outil de préparation osseuse, tel qu'un foret de perçage ou une broche d'ancrage. On comprend donc que ces trous 44 sont débouchants directement sur le fémur F, sans interférer ni avec la pièce interposée 50, ni avec le support 30. Avantageusement, comme dans l'exemple de réalisation considéré sur les figures, ces trous 44 sont avantageusement bordées par des canons 45, prévus en saillie sur l'un et/ou l'autre des deux faces du corps 41, opposées selon l'axe Z-Z, comme bien visible sur la figure 5. Ces canons 45 renforcent le guidage et le centrage de l'outil de préparation osseuse, dans les trous 44.

Les composants du système 20 ayant été décrits en détail, la suite de la description reprend le cours de l'intervention chirurgicale, dont la première étape avait été décrite en regard des figures 1 et 2. Ainsi, après que le chirurgien a dégagé la partie détachable 14 du bloc 10, comme expliqué plus haut, il se saisit du système mécanique 20 et le fixe sur la partie principale 13 du bloc 10, comme sur les figures 3 à 5. Plus précisément, le chirurgien solidarise le plot 32 avec le collier 17. Ce faisant, le système mécanique 20 se trouve alors positionné par rapport au fémur de sorte que, d'une part, l'axe X-X, défini par son support 30, s'étend suivant une direction anatomique antéro-postérieure et, d'autre part, l'axe Z-Z, défini par sa pièce interposée 50, s'étend selon la direction longitudinale du fémur. Ainsi, on comprend que, avant d'utiliser les trous 44 de la pièce de guidage 40 pour guider l'application d'un outil de préparation osseuse sur la face distale de l'extrémité inférieure du fémur F, le chirurgien a la possibilité d'ajuster librement la position de cette pièce de guidage 40 vis-à-vis du fémur F : plus précisément, moyennant une rotation selon la flèche R autour de l'axe Z-Z, la pièce de guidage 40 peut être ajustée à la rotation externe-interne du fémur F que constate le chirurgien en per-opératoire et, moyennant une translation selon la flèche T de la pièce 50, et par là de la pièce de guidage 40, le long de l'axe X-X, la position de la pièce de guidage 40 peut être ajustée à la translation antéro-postérieure du fémur que constate le chirurgien en per-opératopire. Comme déjà évoqué précédemment dans le présent document, l'ajustement lié à la rotation externe-interne du fémur vise à éviter que les plans de résection, qui vont être réalisés ultérieurement, sur la base d'un positionnement déterminé par la pièce de guidage 40, ne soient pas orientés avec la même valeur de rotation externe-interne que celle constatée du fémur F opéré. En d'autres termes, cela revient à ajuster librement la position angulaire de la pièce de guidage 40, autour de l'axe Z-Z, à la position peropératoire du fémur autour de son axe longitudinal. L'ajustement lié à la translation antéro-postérieure du fémur est pris en compte par le chirurgien selon que ce dernier privilégie le positionnement de l'implantation du composant prothétique fémoral soit par rapport à la corticale antérieure du fémur, soit par rapport au niveau de résection postérieure des condyles du fémur en lien avec l'espace prothétique en flexion, soit par rapport à un compromis entre les deux références de positionnement précitées.

En pratique, le chirurgien s'aide des graduations 34 et 58 pour quantifier l'ajustement en position de la pièce de guidage 40, qu'il réalise au cours de l'intervention. Une fois que cet ajustement correspond à ses attentes, il utilise la mollette 57 et la tige 43 pour bloquer en position la pièce de guidage 40 par rapport au support 30, et par là, par rapport au bloc fémoral 10.

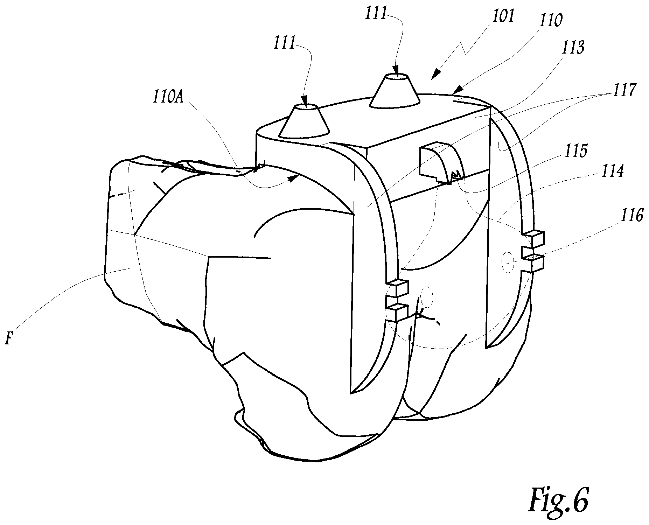

Bien entendu, on comprend que la quantification de l'ajustement en position de la pièce de guidage 40 à la rotation externe-interne du fémur F dépend d'une référence « zéro » prédéterminée. Dans le mode de réalisation considéré sur les figures 1 à 5, cette référence « zéro » est la ligne condylienne postérieure, c'est-à-dire la ligne virtuelle anatomique reliant les faces postérieures des deux condyles de l'extrémité inférieure du fémur F. Ce référencement est imposé par la conception du système mécanique 20, dans le sens où, dans la mesure où la position du bloc fémoral 10 sur le fémur 20 est connue avec précision grâce aux données pré-opératoires utilisées pour fabriquer ce bloc, notamment pour conformer la surface d'appui 10A, la fixation du système mécanique 20 sur le bloc 10, via la coopération entre le collier 17 et le plot 32, peut avantageusement être conçue pour prédéterminer la valeur « zéro » du positionnement relatif angulaire, autour de l'axe Z-Z entre la pièce 40 et le bloc 10, en prenant comme référence la ligne condylienne postérieure. Bien entendu, à titre de variantes, d'autres références que la ligne condylienne postérieure peuvent être envisagées, notamment la ligne de Whiteside ou l'axe transépicondylien ou encore une combinaison de ces références, étant remarqué que cette dernière référence sera évoquée plus en détail lors de la description à venir des figures 6 à 9.

Une fois que cet ajustement en position de la pièce de guidage 40 est réalisé, le chirurgien utilise les trous 44 pour préparer la face distale de l'extrémité inférieure du fémur F, notamment en y mettant en place deux broches, comme indiqué par les flèches A sur la figure 5. En particulier, les broches précitées sont ensuite utilisées, après dégagement de l'instrumentation 1, pour positionner un ou plusieurs blocs de coupe pour réaliser une ou plusieurs résections de l'extrémité inférieure du fémur, selon des plans respectifs prédéterminés, imposés par ce ou ces blocs de coupe.

Sur les figures 6 à 9 est représentée une instrumentation chirurgicale 101, constituant un second mode de réalisation comparé à l'instrumentation 1. L'instrumentation 101a la même finalité que l'instrumentation 1, à savoir, tout en étant spécifique à un patient donné, permettre à un chirurgien de préparer l'extrémité inférieure du fémur de ce patient, notamment en vue de l'implantation du composant fémoral d'une prothèse de genou. L'instrumentation 101 comprend à cet effet des composants fonctionnellement similaires aux composants de l'instrumentation 1, à savoir un bloc fémoral 110 et un système mécanique 120 permettant un ajustement à la rotation externe-interne du fémur F, qui sont respectivement similaires, d'un point de vue fonctionnel, au bloc 10 et au système 20 de l'instrumentation 1.

Comparativement au bloc 10, le bloc fémoral 110 de l'instrumentation 101 présente une surface 110A d'appui sur l'extrémité inférieure du fémur F, qui est similaire à la surface 10A du bloc 10. De plus, suivant des considérations similaires à celles développées plus haut, en lien avec les parties 13 et 14 du bloc 10, le bloc 110 inclut :
- une partie principale 113, qui délimite la majeure partie, voir l'essentiel de la surface d'appui 110A, et qui est pourvue de trous traversants 111 de réception de broche de fixation du bloc 110 sur le fémur F, qui sont fonctionnellement similaires aux trous 11 du bloc 10 ; et
- une partie 114 détachable de la partie 113 par rupture le long d'une ligne de brisure 115, cette partie détachable 114, qui n'est représentée qu'en pointillés sur la figure 6, étant pourvue de trous 116, fonctionnellement similaires aux trous 16 de la partie 14.

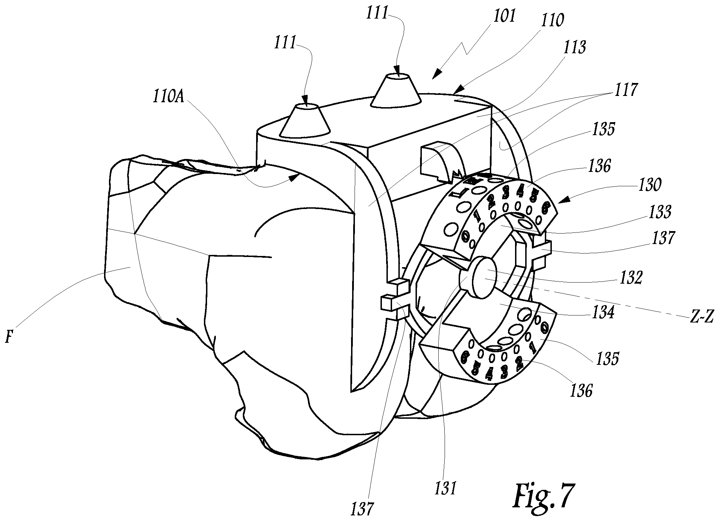

Ainsi, on comprend que le bloc 110 s'utilise, lors du début de l'intervention chirurgicale visant à préparer l'extrémité inférieure du fémur F, de la même façon que le bloc 10. Après que le chirurgien ait accédé au fémur F, il fixe le bloc 110 sur l'extrémité inférieure du fémur, à l'aide de broches introduites dans les trous traversants 111. Puis, pour les raisons évoquées plus haut, il peut décider de dégager la partie détachable 14, en la séparant de la partie principale 113, libérant ainsi un espace libre pour la mise en place du système d'ajustement 120, comme représenté sur les figures 7 à 9.

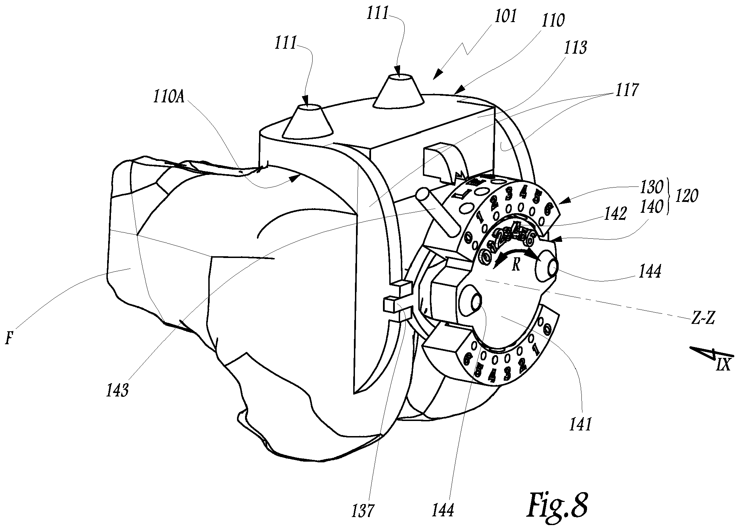

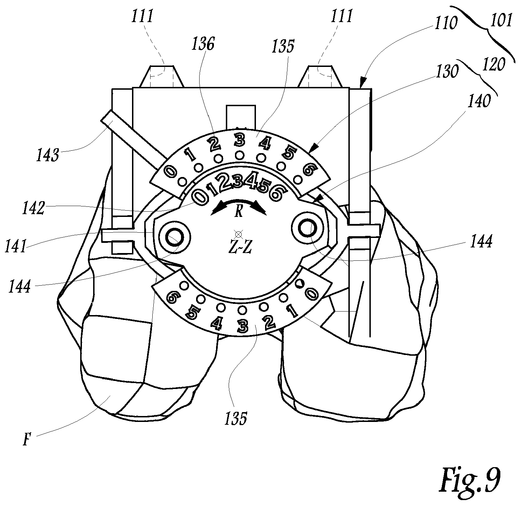

Le système 120 ne comprend, à la différence du système 20 de l'instrumentation 1, que deux composants principaux, à savoir un support 130, représenté seul à la figure 7, et une pièce de guidage 140, montrée en utilisation sur les figures 8 et 9. Ainsi, comme bien visible sur la figure 7, le support 130 comprend un corps rigide 131 incluant un axe physique central 132, qui est centré sur un axe géométrique Z-Z et depuis lequel s'étendent, de manière diamétralement opposée et, avantageusement, en étant symétriques l'une de l'autre, deux portions de disque 133 et 134, centrées sur l'axe Z-Z. Chacune des portions de disque 133 et 134 est structurellement similaire à la portion de disque 54 du système 20, notamment en incluant chacune une bordure périphérique 135 portant des graduations 136, respectivement similaires à la bordure 59 et aux graduations 58.

Le corps 131 du support 130 est en outre pourvu de deux étriers rigides 137, qui sont diamétralement opposés l'un à l'autre vis-à-vis de l'axe Z-Z, en étant situés dans les cadrans non occupés par les portions de disque 133 et 134. Chaque étrier 137 présente une forme globalement « Y », dont l'intérêt apparaîtra plus loin et dont les branches s'étendent en saillie depuis les deux extrémités, en regard, de la périphérie des deux portions de disques 133 et 134. Ces deux étriers 137 sont conçus pour permettre la fixation du support 130 sur le bloc 110, de manière fonctionnellement similaire au plot 32 du support 30. Pour ce faire, la partie principale 113 du bloc 110 est pourvue de deux bras 117 de couplage respectif des étriers 137 : l'intérêt de la forme de ces bras 117 et de ces étriers 137 apparaîtra un peu plus loin.

Comme représenté sur les figures 8 et 9, la pièce de guidage 140 comprend un corps rigide 141 qui présente globalement une forme discoïdale, centrée sur l'axe Z-Z. Ce corps discoïdal 141 est conçu pour être monté directement sur le support 130, en s'engageant de manière librement rotative autour de son axe physique 132, de manière similaire au montage du corps 41 sur le moyeu 52 au sein du système 20 de l'instrumentation 1. De manière similaire aux références 42 et à la tige 43 associées à la pièce de guidage 40, le corps 141 de la pièce de guidage 140 porte des références 142 et est conçu pour coopérer, conjointement avec les bordures 135 des portions de disque 133 et 134 du corps 132, avec une tige de blocage 143. De plus, la pièce de guidage 140 est pourvue de deux trous traversants 144 fonctionnellement similaires aux trous 44 de la pièce de guidage 40, notamment en débouchant à la fois dans les cadrans libres séparant les portions de disque 133 et 134 autour de l'axe Z-Z et entre les branches de la forme particulière en « Y » de chaque étrier 137.

Ainsi, on comprend que, à la différence du système 20 de l'instrumentation 1, le système 120 n'autorise, entre le support 130 et la pièce de guidage 140, qu'une rotation relative autour de l'axe Z-Z, sans permettre un mouvement translatif similaire à celui présent au sein du système 20, grâce à la pièce interposée 50.

Concernant le fonctionnement du système 120, il se déduit du système 20, en excluant les considérations liées à l'ajustement en translation entre la pièce 40 et le support 30. Autrement dit, au cours de l'intervention chirurgicale, une fois que le chirurgien a décidé qu'il souhaitait poursuivre l'intervention de préparation du fémur F en se passant du guidage de positionnement fourni par les trous 116, il dégage la partie détachable 114 puis rapporte le système 120 sur le bloc 110, en fixant le support 130 sur la partie principale 113 du bloc 110, par coopération entre les étriers 137 et les bras 117. En pratique, le chirurgien rapporte le support 130 et la pièce 140 sous forme d'un ensemble pré-assemblé, sans nécessairement passer par une étape intermédiaire correspondant à la figure 7 qui n'est fournie ici qu'à titre d'illustration et de compréhension. Puis, selon la rotation autour de l'axe Z-Z que le chirurgien souhaite imposer aux trous de guidage 144, il bascule d'autant la pièce de guidage 140, en quantifiant ce basculement à l'aide des graduations 136 et des références 142, puis en bloquant la position basculée souhaitée à l'aide de la tige 143, comme représenté sur les figures 8 et 9.

Dans ce contexte, on comprend mieux l'intérêt de la forme de réalisation des bras 117 et des étriers 137. En effet, la droite rectiligne que matérialisent les corps principaux respectifs des étriers 137 est conçue pour correspondre à une direction parallèle à l'axe transépicondylien. Bien entendu, ce résultat est obtenu par la conception même du bloc 110 et du support 130, en exploitant les données de cartographie pré-opératoires mesurées sur le fémur F. Dans ce cas, c'est avantageusement l'axe transépicondylien qui est choisi, lors de la conception du système 120, pour prédéterminer la valeur « zéro » du positionnement relatif angulaire, autour de l'axe de rotation Z-Z, entre la pièce de guidage 140 et le bloc fémoral 110 sur lequel est rapporté fixement le support 130.

Divers aménagements et variantes aux instrumentations 1 et 101 décrites jusqu'ici sont par ailleurs envisageables. Par exemple, une variante de réalisation, qui s'applique aussi bien à l'instrumentation 1 qu'à l'instrumentation 101, consiste à prévoir que le bloc fémoral 10 ou 110 soit réalisé d'une seule pièce avec le support 30 ou 130 du système 20 ou 120 : cela revient à dire que l'invention s'applique également à une instrumentation spécifique à un patient, qui intègre, dès sa conception, la présence d'un système mécanique permettant d'ajuster le guidage de l'application per-opératoire sur le fémur d'un outil de préparation osseuse, à la rotation du fémur. Bien entendu, une telle variante induit un surcoût pour l'instrumentation sur mesure correspondante, ce surcoût étant, à l'inverse, amortit pour les modes de réalisation considérés sur les figures dans le sens où les systèmes 20 et 120 présentent l'avantage de pouvoir être réutilisés en étant successivement associés à plusieurs blocs fémoraux 10 et 110 différents, au cours d'autant d'interventions chirurgicales successives.

## Revendications

1. Instrumentation chirurgicale spécifique à un patient (1 ; 101) pour la préparation du fémur du patient,
comportant un bloc fémoral spécifique au patient (10; 110), qui délimite une surface (10A ; 11A) d'appui fixe sur le fémur (F), conformée de manière spécifiquement ajustée au fémur,
**caractérisée en ce que** l'instrumentation comporte également un système mécanique (20 ; 120) d'ajustement à la rotation externe-interne du fémur (F), ce système d'ajustement étant, en service, partiellement lié fixement au bloc fémoral (10 ; 110).

2. Instrumentation suivant la revendication 1, **caractérisée en ce que** le système d'ajustement (20 ; 120) inclut une pièce de guidage (40 ; 140) pour guider l'application per-opératoire sur le fémur (F) d'un outil de préparation osseuse, tel qu'un foret ou une broche.

3. Instrumentation suivant la revendication 2, **caractérisée en ce que** le système d'ajustement (20 ; 120) inclut en outre un support (230 ; 130), qui, en service, est lié fixement au bloc fémoral (10; 110) et auquel la pièce de guidage (40; 140) est assemblée de manière basculante autour d'un axe de rotation (Z-Z) pour ajuster l'application de l'outil à la rotation du fémur (F) sur lui-même autour de son axe longitudinal.

4. Instrumentation suivant la revendication 3, **caractérisée en ce que** le support (30 ; 130) est distinct du bloc fémoral (10 ; 110) et pourvu de moyens (32 ; 137) de fixation sur le bloc (10 ; 110), adaptés pour coopérer avec des moyens de couplage dédiés (17 ; 117) appartenant au bloc.

5. Instrumentation suivant la revendication 4, **caractérisée en ce que** le bloc (10 ; 110) inclut :
- une partie principale (13 ; 113), qui délimite au moins une partie de la surface d'appui fixe (10A ; 110A), qui est adaptée pour être fixée au fémur (F), et qui porte les moyens de couplage (17 ; 117) ; et
- une partie détachable (14 ; 114), à même d'être séparée de la partie principale (13 ; 113) par rupture du lien de matière entre elles, pour dégager un espace libre pour la fixation du support (30 ; 130) sur la partie principale (13 ; 113) du bloc (10 ; 110).

6. Instrumentation suivant la revendication 3, **caractérisée en ce que** le support et le bloc fémoral sont réalisés d'une seule pièce.

7. Instrumentation suivant l'une quelconque des revendications 3 à 6, **caractérisée en ce que** la pièce de guidage (40) est également assemblée au support (30) de manière coulissante le long d'un axe de translation (X-X), sensiblement perpendiculaire à l'axe de rotation (Z-Z), pour ajuster l'application de l'outil à la translation antéro-postérieure du fémur (F).

8. Instrumentation suivant la revendication 7, **caractérisée en ce que** le système d'ajustement (20) inclut en outre une pièce interposée (50) liant mécaniquement l'un à l'autre le support (30) et la pièce de guidage (40), **en ce que** l'axe de translation (X-X) est défini fixement par le support (30), **en ce que** la pièce interposée (50) est montée sur le support (30) de manière coulissante selon l'axe de translation (X-X), **en ce que** l'axe de rotation (Z-Z) est défini fixement par la pièce interposée (50), et **en ce que** la pièce de guidage (40) est montée sur la pièce interposée (50) de manière basculante autour de l'axe de rotation (Z-Z).

9. Instrumentation selon l'une quelconque des revendications 3 à 6, **caractérisée en ce que** l'axe de rotation (Z-Z) est défini fixement par le support (130), et **en ce que** la pièce de guidage (140) est montée sur le support (130) de manière basculante autour de l'axe de rotation (Z-Z).

10. Instrumentation suivant l'une quelconque des revendications 3 à 9, **caractérisée en ce que** le système d'ajustement (20 ; 120) est conçu pour prédéterminer la valeur « zéro » du positionnement relatif angulaire, autour de l'axe de rotation (Z-Z), entre la pièce de guidage (40 ; 140) et le bloc fémoral (10 ; 110), en prenant comme référence la ligne condylienne postérieure.

11. Instrumentation suivant l'une quelconque des revendications 3 à 10, **caractérisée en ce que** le système d'ajustement (20 ; 120) est conçu pour prédéterminer la valeur « zéro » du positionnement relatif angulaire, autour de l'axe de rotation (Z-Z), entre la pièce de guidage (40 ; 140) et le bloc fémoral (10 ; 110), en prenant comme référence l'axe transépicondylien.

12. Instrumentation suivant l'une quelconque des revendications 3 à 11, **caractérisée en ce que** le système d'ajustement (20 ; 120) est conçu pour prédéterminer la valeur « zéro » du positionnement relatif angulaire, autour de l'axe de rotation (Z-Z), entre la pièce de guidage (40 ; 140) et le bloc fémoral (10 ; 110), en prenant comme référence la ligne de Whiteside.

13. Instrumentation suivant l'une quelconque des revendications 3 à 12, **caractérisée en ce que** le système d'ajustement (20 ; 120) est conçu pour prédéterminer la valeur « zéro » du positionnement relatif angulaire, autour de l'axe de rotation (Z-Z), entre la pièce de guidage (40 ; 140) et le bloc fémoral (10 ; 110), en prenant comme référence une combinaison de références parmi la ligne condylienne postérieure, l'axe transépicondylien et la ligne de Whiteside.

14. Instrumentation suivant l'une quelconque des revendications 3 à 13, **caractérisée en ce que** le système d'ajustement (20 ; 120) inclut en outre des graduations (34, 58 ; 136) de quantification de la position de la pièce de guidage (40 ; 140) autour de l'axe de rotation (Z-Z) et, le cas échéant, le long de l'axe de translation (X-X).

15. Instrumentation suivant l'une quelconque des revendications 3 à 14, **caractérisée en ce que** le système d'ajustement (20, 120) inclut en outre des moyens mécaniques de blocage réversible (43, 57 ; 143), adaptés pour immobiliser la position de la pièce de guidage (40 ; 140) autour de l'axe de rotation (Z-Z) et, le cas échéant, le long de l'axe de translation (X-X).

## Patentansprüche

1. Spezifische chirurgische Instrumentation für einen Patienten (1; 101) für die Vorbereitung des Femurs des Patienten, die eine spezifischen femoralen Block für den Patient (10; 110) aufweist, der eine feststehende Stützoberfläche (10A; 11A) auf dem Femur (F) auf eine Weise begrenzt, die spezifisch an das Femur angepasst ist,
**dadurch gekennzeichnet, dass** die Instrumentation ebenfalls ein mechanisches System zur Anpassung (20; 120) an die die interne-externe Rotation des Femurs (F) aufweist, wobei dieses bei der Verwendung teilweise fest mit dem femoralen Block (10; 110) verbunden ist.

2. Instrumentation nach Anspruch 1, **dadurch gekennzeichnet, dass** das System zur Anpassung (20; 120) ein Führungsstück (40; 140) zur Führung der voroperativen Anwendung eines Werkzeugs zur Knochenvorbereitung wie einen Bohrer oder Stift auf dem Femur (F) einschließt.

3. Instrumentation nach Anspruch 2, **dadurch gekennzeichnet, dass** das System zur Anpassung (20; 120) ferner einen Träger (230; 130) einschließt, der bei der Verwendung fest mit dem femoralen Block (10; 110) verbunden ist, und mit welchem das Führungsstück (40; 140) so verbunden ist, dass es um eine Drehachse (Z-Z) schwenkbar ist, um die Anwendung des Werkzeuges an die Drehung des Femurs (F) um sich selbst um seine Längsachse anzupassen.

4. Instrumentation nach Anspruch 3, **dadurch gekennzeichnet, dass** der Träger (30; 130) von dem femoralen Block (10; 110) getrennt ist und mit Mitteln (32; 137) zur Befestigung auf dem Block (10; 110) versehen ist, die angepasst sind, mit dedizierten Kopplungsmitteln (17; 171) zusammenzuwirken, die zu dem Block gehören.

5. Instrumentation nach Anspruch 4, **dadurch gekennzeichnet, dass** der Block (10; 110) einschließt:
- einen Hauptteil (13; 113), der zumindest einen Teil der feststehenden Stützfläche (10A; 110A) abgrenzt, die angepasst ist, an das Femur (F) befestigt zu werden, und welcher die Kupplungsmittel (17; 117) trägt; und
- einen entfernbaren Teil (14; 114), der angepasst, selbst von dem Hauptteil (13; 113) durch einen Bruch der Materialverbindung zwischen ihnen getrennt zu werden, um einen Freiraum für die Befestigung des Trägers (30; 130) auf dem Hauptteil (13; 113) des Blocks (10; 110) freizugeben.

6. Instrumentation nach Anspruch 3, **dadurch gekennzeichnet, dass** der Träger und der femorale Block aus einem einzigen Stück hergestellt sind.

7. Instrumentation nach einem der Ansprüche 3 bis 6, **dadurch gekennzeichnet, dass** das Führungsstück (40) auch an dem Träger (30) verschiebbar entlang einer Translationsachse (X-X) im Wesentlichen senkrecht zu der Drehachse (Z-Z) montiert ist, um die Anwendung des Werkzeuges an die antero-posteriore Translation des Femurs (F) anzupassen.

8. Instrumentation nach Anspruch 7, **dadurch gekennzeichnet, dass** das System zur Anpassung (20) ferner einen Zwischenteil (50) einschließt, der mechanisch miteinander den Träger (30) und das Führungsstück (40) verbindet, dass die Translationsachse (X-X) feststehend durch den Träger (30) definiert ist, dass der Zwischenteil (50) auf dem Träger (30) verschiebbar entlang der Translationsachse (X-X) angebracht ist, dass die Drehachse (Z-Z) feststehend durch den Zwischenteil (50) definiert ist, und dass das Führungsstück (40) auf dem Zwischenteil (50) schwenkbar um die Drehachse (Z-Z) angebracht ist.

9. Instrumentation nach einem der Ansprüche 3 bis 6, **dadurch gekennzeichnet, dass** die Drehachse (ZZ) fest durch den Träger (130) definiert ist, und dass das Führungsstück (140) auf dem Träger (130) schwenkbar um die Drehachse (Z-Z) angebracht ist.

10. Instrumentation nach einem der Ansprüche 3 bis 9, **dadurch gekennzeichnet, dass** das System zur Anpassung (20; 120) ausgelegt ist, den Wert "null" der relativen Winkelpositionierung um die Drehachse (Z-Z) zwischen dem Führungsstück (40; 140) und dem femoralen Block (10; 110) vorzubestimmen, wobei die posteriore kondyläre Linie als Referenz genommen wird.

11. Instrumentierung nach einem der Ansprüche 3 bis 10, **dadurch gekennzeichnet, dass** das System zur Anpassung (20; 120) ausgelegt ist, den Wert "null" der relativen Winkelpositionierung um die Drehachse (Z-Z) zwischen dem Führungsstück (40; 140) und dem femoralen Block (10; 110) vorzubestimmen, wobei die transepikondyläre Achse als Referenz genommen wird.

12. Instrumentation nach einem der Ansprüche 3 bis 11, **dadurch gekennzeichnet, dass** das System zur Anpassung (20; 120) ausgelegt ist, den Wert "null" der relativen Winkelpositionierung um die Drehachse (Z-Z) zwischen dem Führungsstück (40; 140) und dem femoralen Block (10; 110) vorzubestimmen, wobei die Whiteside-Linie als Referenz genommen wird.

13. Instrumentation nach einem der Ansprüche 3 bis 12, **dadurch gekennzeichnet, dass** das System zur Anpassung (20; 120) ausgelegt ist, den Wert "null" der relativen Winkelpositionierung um die Drehachse (Z-Z) zwischen dem Führungsstück (40; 140) und dem femoralen Block (10; 110) vorzubestimmen, wobei eine Kombination von Referenzen aus der posterioren kondylären Linie, der transepikondylären Achse und der Whiteside-Linie als Referenz genommen wird.

14. Instrumentation nach einem der Ansprüche 3 bis 13, **dadurch gekennzeichnet, dass** das System zur Anpassung (20; 120) ferner Abstufungen (34, 58; 136) zur Quantifizierung der Position des Führungsstücks (40; 140) um die Drehachse (Z-Z) und, falls erforderlich, entlang der Translationsachse (X-X) einschließt.

15. Instrumentation nach einem der Ansprüche 3 bis 14, **dadurch gekennzeichnet, dass** das System zur Anpassung (20, 120) ferner mechanische Mittel zur reversiblen Verriegelung (43, 57; 143) einschließt, die angepasst sind, um die Position des Führungsstücks (40; 140) um die Drehachse (Z-Z) und, gegebenenfalls entlang der Translationsachse (X-X), zu immobilisieren.

## Claims

1. Patient-specific surgical instrumentation (1; 101) for preparing the patient's femur, including a patient-specific femoral block (10; 110), which defines a fixed bearing surface (10A; 11A) on the femur (F), configured to be specifically adjusted to the femur,
but **characterized in that** the instrumentation also includes a mechanical system (20; 120) for adjusting to the external-internal rotation of the femur (F), this adjustment system being, during use, partially fixedly connected to the femoral block (10; 110).

2. The instrumentation according to claim 1, **characterized in that** the adjustment system (20; 120) includes a guide piece (40; 140) to guide the perioperative application on the femur (F) of a bone preparation tool, such as a drill or a pin.

3. The instrumentation according to claim 2, **characterized in that** the adjustment system (20; 120) further includes a support (230; 130), which, during use, is fixedly connected to the femoral block (10; 110) and to which the guide piece (40; 140) is assembled so as to tilt around a rotation axis (Z-Z) to adjust the application of the tool to the rotation of the femur (F) on itself around its longitudinal axis.

4. The instrumentation according to claim 3, **characterized in that** the support (30; 130) is separate from the femoral block (10; 110) and provided with means (32; 137) for fastening on the block (10; 110), suitable for cooperating with dedicated coupling means (17; 117) belonging to the block.

5. The instrumentation according to claim 4, **characterized in that** the block (10; 110) includes:
- a main part (13; 113), which defines at least part of the fixed bearing surface (10A; 110A), which is suitable for being fastened to the femur (F), and which bears the coupling means (17; 117); and
- a detachable part (14; 114), able to be separated from the main part (13; 113) by breaking of the material connections between them, to free a free space to fasten the support (30; 130) on the main part (13; 113) of the block (10; 110).

6. The instrumentation according to claim 3, **characterized in that** the support and the femoral block are made in a single piece.

7. The instrumentation according to any one of claims 3 to 6, **characterized in that** the guide piece (40) is also assembled to the support (30) so as to slide along a translation axis (X-X), substantially perpendicular to the rotation axis (Z-Z), to adjust the application of the tool to the anteroposterior translation of the femur (F).

8. The instrumentation according to claim 7, **characterized in that** the adjustment system (20) further includes an interposed piece (50) mechanically connecting the support (30) and the guide piece (40) to one another, **in that** the translation axis (X-X) is fixedly defined by the support (30), **in that** the interposed piece (50) is mounted on the support (30) so as to slide along the translation axis (X-X), **in that** the rotation axis (Z-Z) is defined fixedly by the interposed piece (50), and **in that** the guide piece (40) is mounted on the interposed piece (50) so as to tilt around the rotation axis (Z-Z).

9. The instrumentation according to any one of claims 3 to 6, **characterized in that** the rotation axis (Z-Z) is defined fixedly by the support (130), and **in that** the guide piece (140) is mounted on the support (130) so as to tilt around the rotation axis (Z-Z).

10. The instrumentation according to any one of claims 3 to 9, **characterized in that** the adjustment system (20; 120) is designed to predetermine the "zero" value ofthe relative angular position, around the rotation axis (Z-Z), between the guide piece (40; 140) and the femoral block (10; 110), using the posterior condylar line as reference.

11. The instrumentation according to any one of claims 3 to 10, **characterized in that** the adjustment system (20; 120) is designed to predetermine the "zero" value ofthe relative angular position, around the rotation axis (Z-Z), between the guide piece (40; 140) and the femoral block (10; 110), using the transepicondylar axis as reference.

12. The instrumentation according to any one of claims 3 to 11, **characterized in that** the adjustment system (20; 120) is designed to predetermine the "zero" value ofthe relative angular position, around the rotation axis (Z-Z), between the guide piece (40; 140) and the femoral block (10; 110), using the Whiteside line as reference.

13. The instrumentation according to any one of claims 3 to 12, **characterized in that** the adjustment system (20; 120) is designed to predetermine the "zero" value ofthe relative angular position, around the rotation axis (Z-Z), between the guide piece (40; 140) and the femoral block (10; 110), using a combination of references from among the posterior condylar line, the transepicondylar axis and the Whiteside line as reference.

14. The instrumentation according to any one of claims 3 to 13, **characterized in that** the adjustment system (20; 120) further includes graduations (34, 58; 136) for quantifying the position of the guide piece (40; 140) around the rotation axis (Z-Z) and, if applicable, along the translation axis (X-X).

15. The instrumentation according to any one of claims 3 to 13, **characterized in that** the adjustment system (20; 120) further includes reversible mechanical blocking means (43, 57; 143), suitable for immobilizing the position of the guide piece (40; 140) around the rotation axis (Z-Z) and, if applicable, along the translation axis (X-X).
